# EUROPEAN PATENT SPECIFICATION

(11) **EP 3 208 335 B1**
(45) Date of publication and mention of the grant of the patent: **01.07.2020**
(21) Application number: 14903871.3
(22) Date of filing: 14.10.2014
(51) Int. Cl.: C12N 15/10, C12N 15/11

(54) **METHOD FOR BREAKING NUCLEIC ACID AND ADDING ADAPTOR BY MEANS OF TRANSPOSASE, AND REAGENT**
VERFAHREN ZUM AUFBRECHEN VON NUKLEINSÄURE UND ZUM HINZUFÜGEN EINES ADAPTERS MITTELS TRANSPOSASE UND REAGENZ
PROCÉDÉ DE RUPTURE D'ACIDE NUCLÉIQUE ET D'AJOUT D'ADAPTATEUR AU MOYEN D'UNE TRANSPOSASE, ET RÉACTIF

(43) Date of publication of application: 23.08.2017
(73) Proprietor: MGI Tech Co., Ltd., Yantian District Shenzhen Guangdong 518083 (CN)
(72) Inventor: GENG, Chunyu, Shenzhen Guangdong 518083 (CN); CHEN, Ruoying, Shenzhen Guangdong 518083 (CN); GUO, Rongrong, Shenzhen Guangdong 518083 (CN); ALEXEEV, Andrei, Woodland, California 95776 (US); ZHANG, Yingxin, Mountain View, California 94040 (US); JIANG, Hui, Shenzhen Guangdong 518083 (CN); ZHANG, Wenwei, Shenzhen Guangdong 518083 (CN)
(74) Representative: Hamer, Christopher K.
(86) International application number: PCT/CN2014/088542
(87) International publication number: WO 2016/058127

(56) References cited:
- WO-A1-2012/103545
- WO-A1-2013/131962
- CN-A- 1 145 641
- CN-A- 101 125 873
- CN-A- 102 264 914
- CN-A- 102 703 426
- CN-A- 102 943 074
- CN-A- 103 710 323
- US-A1- 2010 120 098

## Description

### TECHNICAL FIELD

The present disclosure relates to the field of molecular biology and, more particularly, to a method for breaking a nucleic acid and adding an adaptor by means of a transposase, and a reagent.

### BACKGROUND OF THE INVENTION

Since the pyrophosphate sequencing method invented by Roche, which has opened up the next generation of sequencing, until now, the next generation of sequencing has undergone a period of rapid development. However, with the development of high-throughput sequencing, the sample preparation with high-throughput and low-cost has become a key consideration in the field of sequencing. Sample processing methods and automation devices of various principles have been developed, including: samples fragmentation, terminal treatment of nucleic acid molecules and adapters ligation and the generation of final libraries.

The methods of samples fragmentation mainly include physical methods (such as ultrasound shear) and enzymatic methods (i.e., treatment of non-specific endonuclease). Wherein the physical methods are dominated by Covaris based on patented Adaptive Focused Acoustic (AFA) technology. Under an isothermal condition, the acoustic energy with a wavelength of 1 mm is focused on a sample by a spherical solid state ultrasonic sensor with >400kHz, using geometric focusing acoustic energy. This method ensures the integrity of nucleic acid samples, and a high recovery rate can be achieved. Covaris's instruments include an economical M-series, a single-tube full-power S-series and higher-throughput E- and L-series. The randomization of fragments based on physical methods is good, but the physical methods depend on a large number of Covaris interrupters, and require subsequent separate terminal treatment, adapter ligation and PCR, and various purification operations. Wherein the enzymatic methods include the NEB Next dsDNA Fragmentase from NEB company. The reagent first cleaves the double stranded DNA to produce a random cleavage site, and then clears the complementary DNA strand by identifying the cleavage site through another enzyme to achieve the purpose of interruption. This reagent can be used for genomic DNA, whole genome amplification products and PCR products, and randomness is also good, but some artificial short fragments insertion and deletion will be generated. And also inevitably need to carry out subsequent separate terminal treatment, adapter ligation and PCR, and various purification operations. In addition, the transposase disrupting kit led by Nextera kit of Epicentra company (acquired by Illumina) has been used to complete the DNA fragmentation and the adapters ligation simultaneously using the transposase, thereby reducing the time of sample processing.

From the simplicity of the various operations, the method of interruption by transposase is far superior to other methods in terms of flux and ease of operation, but this interruption has its own shortcomings: the transposase's founction is dependent on a specific 19bp Me sequence. Thus, although the transposase can add different adapter sequences at the 5' and 3' ends of the target sequence by embedding two completely different adapter sequences, the adapters need to contain a specific sequence of Me, resulting in a results that both ends of the interrupted fragment will symmetrically have a Me sequence, and due to the special effect of the transposase so that a 9nt base missing gap will present between the target sequence (or the interrupted fragment) and the Me sequence. The identical Me sequences at both ends of the target sequence will have an impact on downstream technology applications, such as an impact on the second-generation sequencing technique based on the ligation method, where the Me sequences on both sides of the same chain are complementary sequences, thus internal annealing of the single-strand molecule will generate and harm the binding of anchoring primers.

There has been a related patent application (Application Publication No.: CN 102703426 A, filed on October 3, 2012) to propose a technical solution, in which an endonuclease digestion is performed on the interrupted sequences to remove the 9nt sequence and the Me sequence. However, this method only uses the advantage of transposase interruption to randomize the nucleic acid sequences, but introduction of a shortcoming that a follow-up adapter needed to be added separately, which is steps-cumbersome and not suitable for higher throughput applications.

So far, there has been no molecular biology experiment method be disclosed by any patents and other literatures to rapidly interrupt the target sequences by the use of transposase technology and to modify the interrupted sequence to two completely different sequences.

### SUMMARY OF THE INVENTION

A method and a reagent for breaking a nucleic acid and adding an adaptor by means of a transposase are provided in the present disclosure, in which other sequences different from the transposase identification sequence are introduced into the nucleic acid product interrupted by the transposase, so as different adapters are ligated to both ends of the interrupted nucleic acid, thus the application of the interrupted product is not limited by the presence of the transposase identification sequence at both ends.

According to a first aspect of the present disclosure, a method for breaking a nucleic acid and adding an adapter by means of a transposase is provided, wherein the method comprises the following steps:
randomly interrupting a nucleic acid by using a transposase-embedded complex, wherein the transposase-embedded complex comprises a transposase and a first adapter comprising a transposase identification sequence, and both ends of the interrupted nucleic acid are separately ligated to the first adapter to form a gap at each end;
eliminating the influence of the transposase in the system on a follow-up reaction by means of purification or chemical reagent treatment;
ligating to a second adapter at the gap by using a ligase, wherein the sequence of the second adapter is different from that of the first adapter; and
performing a PCR reaction by using primers targeted to the first adapter and the second adapter respectively, so as to obtain a product whose both ends are respectively ligated to different adapter sequences.

As a preferred embodiment of the present disclosure, in order to prevent self-ligation or inter-ligation of the adapters, the first adapter having a modification to prevent self-ligation or a modification to ligate with the second adapter.

As a preferred embodiment of the present disclosure, the modification on the first adapter comprises any one of the following or combination thereof:
(a) the 3' terminal base of the first adapter dideoxy modification;
(b) introducing a dUTP into a chain of the first adapter for subsequent enzymatic cleavage of excess adapters;
(c) introducing a base pair at the outside of the transposase identification sequence of the first adapter, wherein the 3' terminal base dideoxy modification; and
(d) the first adapter consisting of a complete sequence, internally complementary to form a 3'-5' phosphodiester bond cross-linked double stranded sequence.

It is to be noted that any modification of (a) to (d) may be used alone or in combination of two or more modifications, and in particular, the modification (a) may be carried out in combination with modifications (b), (c) or (d) separately, in order to achieve a better effect of preventing self-ligation or inter-ligation of the adapters.

As a preferred embodiment of the present invention, the modification on the first adapter is the 3' terminal base of the first adapter dideoxy modification.

As a preferred embodiment of the present invention, in order to prevent the self-ligation of the adapter, the second adapter has a modification preventing self-ligation.

As a preferred embodiment of the present invention, the modification on the second adapter is a 3' terminal base dideoxy modification.

In the present invention, the term " self-ligation " refers to the ligation between different molecules of the same adapter, such as the ligation between different molecules of the first adapter or the ligation between the different molecules of the second adapter; the term " inter-ligation " refers to the ligation between molecules of different kinds of adapters, such as the ligation between the molecules of the first adapter and the molecules of the second adapter.

As a preferred embodiment of the present invention, in order to facilitate the acquisition of single-stranded molecules after PCR reactions for subsequent single-stranded molecular manipulation experiments, one of the primers used in the PCR reaction is a terminal biotin-labeled primer for obtaining single-stranded molecules by biotin-streptavidin affinity reaction. Specifically, after the PCR reaction, the single-stranded molecule with a biotin at the end is separated by binding to a streptavidin on the surface of the magnetic bead.

As a preferred embodiment of the present invention, the purification is purification by magnetic beads or a column. The purification by magnetic beads or a column can completely remove the transposase in the system. In one embodiment of the present disclosure, Ampure XP beads were used for magnetic beads purification, and a column purification was performed using a QIAGEN PCR purification column. There is no doubt that any similar products for magnetic beads purification or column purification can be used in the present invention.

As a preferred embodiment of the present invention, the chemical reagent treatment is a treatment to dissociate the transposase from a target sequence by degenerating or digesting the transposase. Since the transposase belongs to a protein in chemical form, it can be dissociated from the target sequence using a corresponding denaturation or digestion means, although the transposase after this treatment may still be present in the system but has lost its biological activity, thus the follow-up reactions will not be negatively impacted.

As a preferred embodiment of the present invention, the chemical reagent comprises a first reagent and a second reagent; wherein the first reagent comprises one or more members of the group consisting of a protease solution, a SDS solution and a NT buffer for breaking the adsorption effect of the transposase and the target sequence of the nucleic acid; the second reagent comprises a Triton-X100 solution for weakening the influence of the first reagent on the subsequent enzymatic reactions.

In general, the first reagent is first used for treatment followed by the second reagent. The first reagent is used to treat the reaction product of the nucleic acid after the interruption by the transposase so as to break the adsorption effect of the transposase and the target sequence of the nucleic acid, instead of the steps of magnetic beads purification or column purification which is traditional complex and costly. And then the second reagent is used for treatment to weaken the influence of the first reagent on the subsequent enzymatic reactions, ensuring that downstream PCR amplification proceeds smoothly.

It is to be noted that the first reagent may be one or more members of the above solutions, wherein more of the above solutions may be two or three above solutions, such as the protease solution and the SDS solution, the SDS solution and the NT buffer, the protease solution and the NT buffer, the protease solution, the SDS solution and the NT buffer, wherein the NT buffer can be the NT buffer in S5 series of Truprep kit.

As a preferred embodiment of the present invention, ethylenediaminetetraacetic acid (EDTA) is further added for treatment after the treatment with the first reagent, if the first reagent comprises a protease solution. EDTA inhibits protease activity and thus prevents proteases from degrading enzymes in subsequent PCR reactions.

As a preferred embodiment of the present invention, the second reagent comprises Triton-XlOO solution. Triton-XlOO, whose chemical name octylphenyl polyoxyethylene ether, as a nonionic surfactant, in the role of the present invention is to weaken the influence of the first reagent on the subsequent enzymatic reactions.

As a preferred embodiment of the present invention, the second reagent further comprises a Tween-20 solution if the first reagent comprises an SDS solution. The addition of Tween-20 could further weaken the influence of SDS on the subsequent enzymatic reaction and enhance the PCR effect. It should be noted that Tween-20 may be used as a component of the second reagent in the form of a mixture with Triton-X100; it may also be provided separately in the form of separation from Triton-X100, in which case the second reagent refers to the Triton-X100 solution and the Tween-20 solution.

It is to be understood that the first reagent and the second reagent in the present invention are not intended to be limited to a single object or a combination of a plurality of objects. Also, in the present invention, concepts such as "first" and "second", which are used in any case, should not be construed as having the meaning of order or technique, instead their role in the present invention is to distinguish themselves from other objects.

In the present invention, the working concentration of the first reagent and the second reagent can be determined empirically by those skilled in the art. In general, in the first reagent, the working concentration of the protease is preferably from 50 to 5000 mAU / mL, more preferably from 75 to 3750 mAU / mL, most preferably 1500 mAU / mL; the working concentration of EDTA is preferably from 1 to 50mmol / L, more preferably 14 mmol / L; the working concentration of SDS is preferably from 0.01% to 1.5% (by volume), more preferably 1% (by volume); the final concentration of NT buffer can be used according to 1×. In the second reagent, the working concentration of Triton-XlOO is preferably from 0.1% to 2% (by volume), more preferably 1% (by volume); the working concentration of Tween-20 is preferably from 0.1% to 2% (by volume), more preferably 0.5% (by volume).

In the present invention, the sequence of the second adapter is not limited and may be any sequence as long as it is different from the sequence of the first adapter.

As a preferred embodiment of the present invention, the reagent further comprises a second adapter component for ligation into the gap formed by ligating the first adapter to the interrupted nucleic acid at both ends.

According to a second aspect of the present invention, a reagent for breaking a nucleic acid and adding an adapter by means of a transposase is provided, wherein the reagent comprises the following components:
a transposase and a first adapter comprising a transposase identification sequence for forming a transposase-embedded complex to randomly interrupt a nucleic acid, so as both ends of the interrupted nucleic acid are separately ligated to the first adapter to form a gap at each end;
a second adapter and a ligase component for ligating the second adapter at the gap; and
primers targeted to the first adapter and the second adapter respectively, so as to obtain a product whose both ends are respectively ligated to different adapter sequences by performing a PCR reaction.

As a preferred embodiment of the present invention, the first adapter has a modification to prevent self-ligation or a modification to ligate with the second adapter.

As a preferred embodiment of the present disclosure, the modification on the first adapter comprises any one of the following or combination thereof:
(a) the 3' terminal base of the first adapter dideoxy modification;
(b) introducing a dUTP into a chain of the first adapter for subsequent enzymatic cleavage of excess adapters;
(c) introducing a base pair at the outside of the transposase identification sequence of the first adapter, wherein the 3' terminal base dideoxy modification; and
(d) the first adapter consisting of a complete sequence, internally complementary to form a 3'-5' phosphodiester bond cross-linked double stranded sequence.

As a preferred embodiment of the present invention, the second adapter has a modification preventing self-ligation; the modification on the second adapter is a 3' terminal base dideoxy modification.

As a preferred embodiment of the present invention, one of the primers used in the PCR reaction is a terminal biotin-labeled primer for obtaining single-stranded molecules by biotin-streptavidin affinity reaction.

The method of the present invention modifies the sequence by ligating a second adapter on both sides of the product interrupted by a transposase to achieve a different specific sequence on both sides of the the final interrupted product or the PCR product, thus the application of the interrupted product is not limited by the presence of the transposase identification sequence (19bp Me) at both ends, and the application is more flexibility, such as molecular cyclization, digestion or ligation.

### BRIEF DESCRIPTION OF THE DRAWINGS

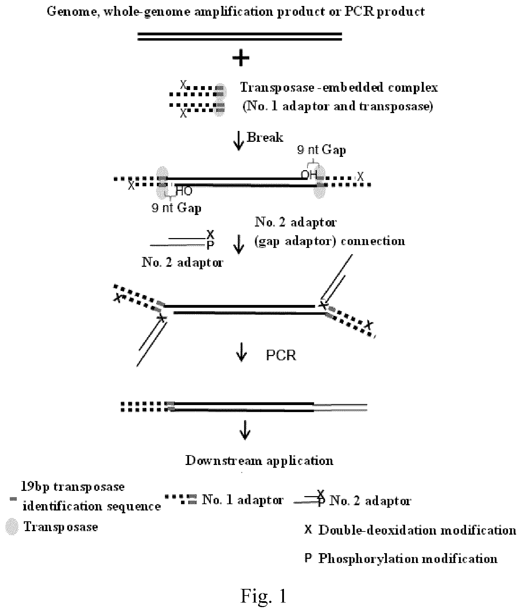

Figure 1 is a schematic flow diagram of a technical solution in which a transposase interrupting a nucleic acid and ligating a gap adapter (i.e., No. 2 adapter) in the present invention;

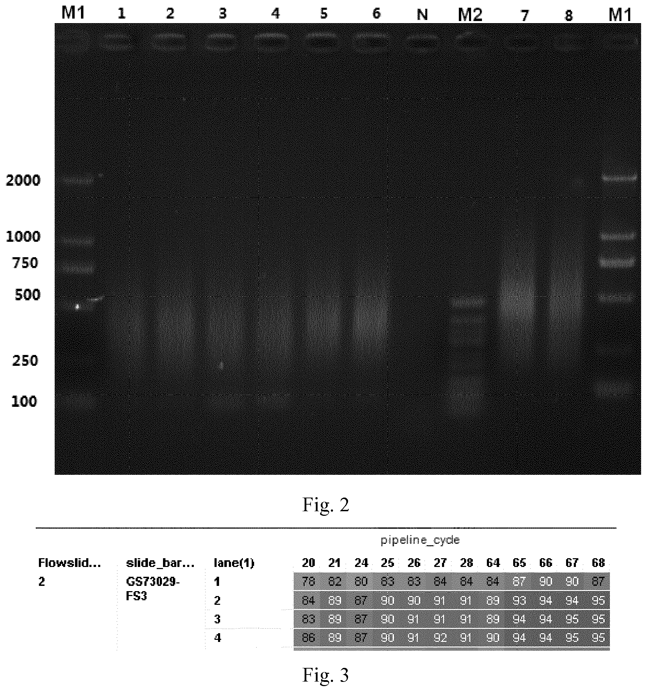

Figure 2 is a result of the gel electrophoresis of the PCR product after the ligation of a gap adapter (i.e., No. 2 adapter) in Example 1 of the present invention, wherein lane 1 is the annealing product at 60 °C after the interruption by single-adapter-2 and after the ligation of the gap adapter; lane 2 is the annealing product at 55°C after the interruption by single-adapter-2 and after the ligation of the gap adapter; lane 3 is the annealing product at 60 °C after the interruption by single-adapter-3 and after the ligation of the gap adapter; lane 4 is the annealing product at 55°C after the interruption by single-adapter-3 and after the ligation of the gap adapter; lane 5 is the annealing product at 60 °C after the interruption by single-adapter-1 and after the ligation of the gap adapter; lane 6 is the annealing product at 55°C after the interruption by single-adapter-1 and after the ligation of the gap adapter; lane 7 is the annealing product at 60 °C after the interruption by double-adapters and after the direct PCR; lane 8 is the annealing product at 55°C after the interruption by double-adapters and after the direct PCR; M1 is the DL2000 DNA Marker; M2 is the 50bp DNA Marker; N is the negative control.
Figure 3 is a base quality diagram by the sequencing of ligation method in Example 1 of the present invention;

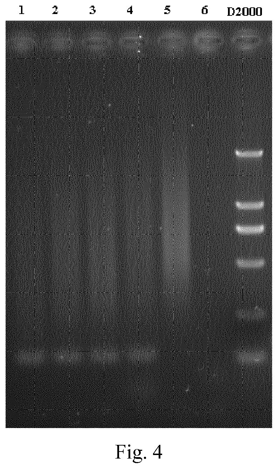

Figure 4 is a result of the gel electrophoresis of the PCR product after the No.1 adapter single-adapter transposase complex interrupting a nucleic acid and after the introduction of the No. 2 adapter in Example 1 of the present invention, wherein D2000 is the lane of DNA Ladder; lane 1 is the result after treatment of 2µL protease +1% Triton-X100; lane 2 is the result after treatment of NT buffer+1% Triton-X100; lane 3 is the result after treatment of 1% SDS +1% Triton-X100+0.5% Tween-20; lane 4 is the result after treatment of 2µL protease +14mM EDTA+1% Triton-XlOO; lane 5 is the result after treatment of 1×PBI, 1.3×Ampure XP beads; lane 6 is the result of a negative control (without template).

### DETAILED DESCRIPTION OF THE INVENTION

The invention will now be described in further detail by way of specific examples. Unless otherwise specified, the techniques used in the examples below are conventional techniques known to those skilled in the art; the instruments and reagents used are accessable to those skilled in the art through public approaches such as commercial approaches and so on.

The terms used in the present invention are set forth as follows: the first adapter is referred to as a No.1 adapter in a specific embodiment; the second adapter is referred to as a No. 2 adapter or gap adapter in a specific embodiment; the first reagent is referred to as a No. 1 reagent in a specific embodiment; and the second reagent is referred to as a No. 2 reagent in a specific embodiment.

Referring to Figure 1, the operation flow of the method of the present invention mainly comprises: (1) a NO. 1 adapter where a specific modification sequence is embedded by a transposase is used to randomly interrupte nucleic acid sequences, such as genomic sequences, whole genome amplification sequences, or PCR product sequences, etc, wherein both ends of the interrupted DNA are ligated to the first adapter and form a 9nt base deletion gap; (2) eliminating the influence of the transposase in the system on a follow-up reaction by means of purification or chemical reagent treatment; (3) introducing a No. 2 adapter by a way of ligating the No. 2 adapter at the 9nt base deletion gap, so as the adapter base sequence adjacent to fragmented target sequence is changed, so that the sequences on both sides of the target sequence are completely different, wherein one remains the NO. 1 adapter sequence containing the transposase identification sequence, while the other is a second adapter completely arbitrarily designed.

In the present disclosure, a transposase kit of domestic production (S50 series of Truprep kit of Nanjing Nuoweizan Ltd.) was used to carry out the following experiment. The kit containes two doses respectively for 5ng genomic DNA and 50ng genomic DNA.

A variety of adapter sequences (the NO. 1 adapter) for embedding was designed in the present disclosure, and a transposase and said adapter sequences for embedding were used to prepare the transposase complex.

### Example 1

In this example, 5ng or 50ng of high quality genomic DNA was first interrupted by the embedded transposase complex; the free unembedded No. 1 adapters were removed after purification by magnetic beads or column purification; then a No. 2 adapter (a gap adapter) was inventively ligated, and the free No. 2 adapters were removed by purification, and thus a linear genome sequence with different adapter sequences at both ends were constructed; a PCR amplification was performed by using PCR primers targeted respectively to the No. 1 adapter and the No. 2 adapter, enriching the PCR product with different adapter sequences at both ends.

One application of the PCR product of this example is by labeling the PCR primers in a biotin-labeled manner, and a single-stranded molecule of a particular sequence is obtained, and a single-stranded cyclic molecule is prepared by a single-stranded cyclization or by a cyclization with a short nucleic acid sequence as a bridge-mediated sequence. The formed single-stranded cyclic molecule can be used for the preparation of solid dense DNA nanospheres.

Multiple pairs of primer sequences (Sequence A and sequence B) with a 19bp transposase identification sequence were designed and manufactured, for the preparation of a single-adapter (the No. 1 adapter) for embedding, and three different single-adapters (i.e., single-adapter 1 sequence, single-adapter 2 sequence and single-adapter sequence) and a standard double-adapters sequence (Sequence A + sequence B; sequence A + sequence C) were tested in the present example.

Wherein a dUTP is introduced into a strand (strand A) of the single-adapter 1 sequence for subsequent digest of excess adapters; a base pair is introduced into the outside of the 19bp transposase identification sequence of the single-adapter 2 sequence, wherein the 3' end base is a dideoxy-modified base; the whole double-stranded sequence of the single-adapter 3 sequence consists of a complete sequence, which is internally complementary to form a double-stranded sequence crosslinked by a 3'-5' phosphodiester bond. In addition, the modification modes of the above-mentioned three kinds of adapters have at least one strand containing a 3'-end dideoxy modification, which helps to prevent the self-ligation of the No. 1 adapter and inter-ligation with the No. 2 adapter. Each of the first adapters sequences is shown as follows:
Sequence A of single-adapter 1:
   CTGTC**U**CTTA**U**ACACATC **ddT** (SEQ ID NO: 1);
Sequence B of single-adapter 1:
   GCTTCGACTGGAGACAGATGTGTATAAGAGACAG (SEQ ID NO: 2);
Sequence A of single-adapter 2:
   **G**CTGTCTCTTATACACATC **ddT** (SEQ ID NO: 3);
Sequence B of single-adapter 2:
   GCTTCGACTGGAGACAGATGTGTATAAGAGACAG **ddC** (SEQ ID NO: 4);
Sequence of single-adapter 3: 
GCTTCGACTGGAGACAGATGTGTATAAGAGACAGCTGTCTCTTATAC
ACATC ddT (SEQ ID NO: 5);
Sequence A of double-adapters:
   CTGTCTCTTATACACATCT (SEQ ID NO: 6);
Sequence B of double-adapters:
   TCGTCGGCAGCGTCAGATGTGTATAAGAGACAG (SEQ ID NO: 7);
Sequence C of double-adapters:
   GTCTCGTGGGCTCGGAGATGTGTATAAGAGACAG (SEQ ID NO: 8).

2. Each pair of single-adapter sequence was diluted to 100µM, fully mixed and centrifuged, annealing to form NO. 1 adapter (stored at -20 °C) in a PCR instrument according to the following procedure (Table 1), for the preparation of embedded complex. The sequence A, B and C of double-adapters were diluted to 100µM, sequence A + sequence B combined, sequence A + sequence C combined, fully mixed and centrifuged, annealing to form NO. 1 adapter (stored at -20 °C) in a PCR instrument according to the following procedure (Table 1), for the preparation of embedded complex.

**Table 1**

| Temperature | Time |
|---|---|
| 75 °C | 15min |
| 60 °C | 10min |
| 50 °C | 10min |
| 40 °C | 10min |
| 25 °C | 30min |
| Hot-lid 105°C | |

3. The NO. 1 adapter and the transposase were embedded into a transposase-embedded complex according to the following system (Table 2), after gently blowing 20 times and incubating 1 hour at 30 °C, the complex embedding was completed. The complex was stored at -20 °C.

**Table 2**

| Component | Content |
|---|---|
| Transposase | 85µL |
| NO. 1 adapter | 30µL |
| Coupling buffer | 85µL |
| Total | 200µL |

4. 50ng of high quality genome and transposase complex were mixed according to the following system (Table 3), after gently mixing 20 times and incubating for 10 minutes at 55°C, and then cooling to 4°C, genome interruption is completed.

**Table 3**

| Component | Content |
|---|---|
| Water | 5µL |
| 5× interruption buffer | 2µL |
| gDNA (50ng/µL) | 1µL |
| Transposase complex | 2µL |
| Total | 10µL |

5. Purification was carried out according to the following two methods. Method 1: A 1-fold volume of PBI (Qiagen PCR Purification Kit) was added and mixed evenly, and purified with 1.3-fold Ampure XP beads (automated operation); Method 2: Purification with QIAGEN PCR Column. After purification, the product was dissolved with pure water.
6. As for the single-adapter 1, after the interruption, USER enzyme was added to digest, and then a purification was carried out similarly to the previous steps, and the reaction system as follows (Table 4):

**Table 4**

| Component | Content |
|---|---|
| DNA | 10µL |
| 10 × Buffer | 2µL |
| USER enzyme | 1µL |
| Water | 7µL |
| Total | 20µL |

7. The product after purification is submitted to the ligation of a gap adapter (i.e., No. 2 adapter) in accordance with the following system (Table 5), the ligation was completed after incubation for 60 minutes at 25 °C.

**Table 5**

| Component | Content |
|---|---|
| Water | 8µL |
| 3×ligation buffer | 20µL |
| No. 2 adapter (5µM) | 10µL |
| Ligase | 2µL |
| DNA | 20µL |
| Total | 30µL |

| | |
|---|---|
| Note: The sequences of the No. 2 adapter are as follows: Sequence A of the No. 2 adapter: p AAGTCGGAGGCCAAGCGGTCGT ddC (SEQ ID NO: 9); Sequence B of the No. 2 adapter: TTGGCCTCCGACT ddT (SEQ ID NO: 10); wherein p represents a 5' terminal phosphorylation modification and dd represents a 3' end dideoxy modification. | |

8. As for the product after ligation, purification was carried out according to the following two methods. Method 1: A 1-fold volume of PBI (Qiagen PCR Purification Kit) was added and mixed evenly, and purified with 1.3-fold Ampure XP beads (automated operation); Method 2: Purification with QIAGEN PCR Column. After purification, the product was dissolved with pure water.
9. PCR amplification was carried out according to the following PCR reaction system (Table 6) and reaction conditions (Table 7).

**Table 6**

| Component | Content |
|---|---|
| DNA product after purification | 30µL |
| 5× PCR buffer | 10µL |
| 10mM dNTP | 1µL |
| Primer 1 | 2µL |
| Primer 2 | 2µL |
| PCR enzyme | 1 µL |
| Pure water | 4µL |
| Total | 50 µL |

| | |
|---|---|
| Note: the PCR primers are as follows: Primer 1 of single-adapter: AGACAAGCTCGAGCTCGAGCGATCGGGCTTCGACTGGAGAC (SEQ ID NO: 11); Primer 2 of single-adapter: TCCTAAGACCGCTTGGCCTCCGACT (SEQ ID NO: 12); Primer 1 of double-adapters: AATGATACGGCGACCACCGA (SEQ ID NO: 13); Primer 2 of double-adapters: CAAGCAGAAGACGGCATACGA (SEQ ID NO: 14). | |

**Table 7**

| Temperature | Time | Cycle |
|---|---|---|
| 72 °C | 3min | 1 Cycle |
| 98 °C | 30sec | 1 Cycle |
| 98 °C | 10sec | 15 Cycles |
| 60°C/55°C | 30sec | |
| 72 °C | 3min | |
| 72 °C | 5min | 1 Cycle |
| 4°C | ∞ | - |

10. The PCR product test results after ligation of gap adapter (No. 2 adapter) are shown in Figure 2, and the PCR product concentration determination results are as follows (Table 8):

**Table 8**

| Adapter | Single - adapte r-2 | Single - adapte r-2 | Single - adapte r-3 | Single - adapte r-3 | Single - adapte r-1 | Single - adapte r-1 | Negati ve control | Doubl e-adapte rs | Double - adapter s |
|---|---|---|---|---|---|---|---|---|---|
| Anneali ng | 60°C | 55°C | 60 °C | 55°C | 60 °C | 55°C | - | 60 °C | 55°C |
| Product concent ration (ng/µL) | 11.8 | 13.8 | 9.6 | 10.1 | 8.24 | 10.3 | 1.64 | 29.8 | 25.8 |

PCR results show that the method of the present invention has successfully introduced the gap adapter.
11. After single-stranded separation of the PCR product, the target band is submitted to single-stranded cyclization, according to the current common means of sequencing, resulting in single-stranded circular DNA molecules for preparation of DNA nanoball by rolling ring replication on a whole genome sequencing platform and for ligation sequencing. The single-stranded separation and cyclization operation is as follows:
(1) The PCR product was subjected to thermal denaturation at 95°C and then immediately ice bath for 5 min;
(2) 3pmol of single-stranded molecules of the PCR product that were denatured were subjected to single-stranded cyclization according to the following reaction system (Table 9);

**Table 9**

| Component | Content |
|---|---|
| Mediating sequences (20µM) | 20µL |
| Pure water | 15 8.3 µL |
| 1 0×ligation buffer | 35µL |
| 100mM ATP | 3.5µL |
| Ligase | 1.2µL |
| PCR product after denature | 112µL |
| Total | 350µL |

| | |
|---|---|
| Note: Mediating sequences are as follows: Mediating sequence for single-adapter: TCGAGCTTGTCTTCCTAAGACCGC (SEQ ID NO: 15); Mediating sequence for double-adapters: CGCCGTATCATTCAAGCAGAAGAC (SEQ ID NO: 16). | |

(3) The single-strand without cyclization is digested, a reaction system is configured according to the following system (Table 10), after mixing and briefly centrifuging, 20µL was added to the previous reaction system, incubating for 30 minutes at 37°C, followed by purification with 1.8-fold Ampure XP beads to prepare a single-stranded cyclic molecule for sequencing.

**Table 10**

| Component | Content |
|---|---|
| 10× ligation buffer | 3.7µL |
| 20U/µL Exonuclease I | 11.1µL |
| 100U/µL Exonuclease III | 5.2µL |
| Total | 20µL |

12. Sequencing can be carried out from the 5' and 3' ends, and the target fragment with different sequences at both ends has a 19bp transposase identification sequence only at one end, thus avoiding the specific annealing of the 19bp transposase identification sequence at both ends and competition with the sequencing adapters, and thus greatly improve the quality of sequencing, the results shown in Figure 3. The data shown in Figure 3 are mostly between 80 and 90, generally above 75, which is acceptable, whereas the data of the conventional sequencing results with the 19bp transposase identification sequence at both ends are generally not so high, which is even between 30 and 40, indicating that the sequencing probe complementary to the 19bp sequence can be well matched to the sequencing template, that is, to solve the effect of the two 19bp reverse complementary sequences on sequencing.

### Example 2

In this example, 50ng of high quality genomic DNA was first interrupted by an embedded transposase complex, followed by treating with protease, SDS, NT or a composition of protease and EDTA to remove the transposase protein bound to DNA; and then after the ligation of a gap adapter, directly amplified using PCR primers, with a certain concentration of TritonX-100 is added into the PCR reaction system.
1. A pair of primer sequences with a 19bp transposase identification sequence, sequence A and sequence B, were designed and prepared, for preparation of NO. 1 adapter in the form of single-adapter:
Sequence A of the NO. 1 adapter in the form single-adapter:
   TCGTCGGCAGCGTCAGATGTGTATAAGAGACAG (SEQ ID NO: 17);
Sequence B of the NO. 1 adapter in the form single-adapter:
   CTGTCTCTTATACACATC ddT (SEQ ID NO: 18, dd represents a dideoxy modification).

2. The sequence A and sequence B were diluted to 100µM, fully mixed and centrifuged, annealing to form the No. 1 adapter (stored at -20 °C) in a PCR instrument according to the following procedure (Table 11), for the preparation of embedded complex.

**Table 11**

| Temperature | Time |
|---|---|
| 75 °C | 15min |
| 60 °C | 10min |
| 50 °C | 10min |
| 40 °C | 10min |
| 25 °C | 30min |
| Hot-lid 105°C | |

3. The NO. 1 adapter and the transposase were embedded into a transposase-embedded complex according to the following system (Table 12), after gently blowing 20 times and incubating 1 hour at 30 °C, the complex embedding was completed. The complex was stored at -20 °C.

**Table 12**

| Component | Content |
|---|---|
| Transposase | 85µL |
| NO. 1 adapter | 30µL |
| Coupling buffer | 85µL |
| Total | 200µL |

4. 50ng of high quality genome and transposase complex were mixed according to the following system (Table 13), after gently mixing 20 times and incubating for 10 minutes at 55°C, and then cooling to 4°C, genome interruption is completed.

**Table 13**

| Component | Content |
|---|---|
| Water | 5µL |
| 5× interruption buffer | 2µL |
| gDNA (50ng/µL) | 1µL |
| Transposase complex | 2µL |
| Total | 10µL |

5. The sample processing methods after the interruption comprises the following options. Method 1: 0.1-5 µL of protease (750 mAU / mL) was added, in this example preferred 2 µL of protease, and at the same time 0.1 µL protease and 5 µL of protease was tested respectively. Method 2: adding the final concentration of commercial 1 × NT buffer (a matching reagent in Truprep kit S5 series). Method 3: adding 0.01% to 1.5% (by volume) of SDS, preferably 1% (by volume) of SDS in this example, and 0.01% (by volume) and 1.5% (by volume) concentrations were tested separately. Method 4: 0.1-5 µL of protease (750 mAU / mL) was added and then added to a final concentration of 1-50 mM EDTA. This example preferred 2 µL of protease and final concentration of 14 mM EDTA, and at the same time 0.1 µL protease plus 1 mM EDTA and 5µL of protease plus 50 mM EDTA was tested. Method 5: adding 1 times of the volume of PBI (a commercial reagent in Qiagen PCR purification kit), after mixing evenly, purifying with 1.3 times of Ampure XP beads, and dissolving with pure water.
6. In the product after the above treatment, 0.1%-2% (by volume) of Triton-X 100 was added, preferably 1% (by volume) in this example, while 0.1% (by volume) and 2% (by volume) of Triton-X100 was used to test.
7. The product treated with the above Triton-X100 was ligated to a gap adapter (the NO. 2 adapter) according to the following system (Table 14) at 25 °C for 60 minutes, the adapter ligation was completed.

**Table 14**

| Component | Content |
|---|---|
| Water | 8µL |
| 3×ligation buffer | 20µL |
| adapter (5µM) | 10µL |
| Liagase | 2µL |
| DNA | 20µL |
| Total | 30µL |

| | |
|---|---|
| Note: Sequence A of the NO. 2 adapter: 5'-pAAGTCGGAGGCCAAGCGGTCGT ddC-3' (SEQ ID NO: 9); Sequence B of the NO. 2 adapter: 5'-TTGGCCTCCGACT ddT-3' (SEQ ID NO: 10)(p represents phosphorylation modification , dd represents dideoxy modification). | |

8. PCR amplification was carried out according to the following PCR reaction system (Table 15) and reaction conditions (Table 16). For the experimental group with SDS added, a specific concentration of Tween-20 was added to the PCR system to partially increase the efficiency of the PCR. The working concentration of Tween-20 could be adjusted to different, such as 0.1% -2% (by volume), preferably 0.5% (by volume) in this example, while the working concentrations of 0.1% (by volume) and 2% (by volume) was tested.

**Table 15**

| Component | Content |
|---|---|
| Processed DNA samples | 30µL |
| 5× PCR buffer | 10µL |
| 10mM dNTP | 1µL |
| Primer 1 | 2µL |
| Primer 2 | 2µL |
| PCR enzyme (DNA polymerase) | 1µL |
| Pure water | 4µL |
| Total | 50 µL |

| | |
|---|---|
| Note: Primer 1 of the NO. 1 adapter in the form of single-adapter: AGACAAGCTCGAGCTCGAGCGATCGGGATCTACACGACTCACTGATC GTCGGCAGCGTC (SEQ ID NO: 19); Primer 2 of the NO. 1 adapter in the form of single-adapter: TCCTAAGACCGCTTGGCCTCCGACT (SEQ ID NO: 20). | |

**Table 16**

| Temperature | Time | Cycle |
|---|---|---|
| 72 °C | 3min | 1 Cycle |
| 98 °C | 30sec | 1 Cycle |
| 98 °C | 10sec | 15 Cycles |
| 60 °C | 30sec | |
| 72 °C | 3min | |
| 72 °C | 5min | 1 Cycle |
| 4°C | ∞ | - |

9. PCR product detection result of after interruption by single-adapter embedding complex and ligation of the gap adapter is shown in Figure 4, and the PCR product concentration determination results are shown in Table 17.

**Table 17**

| Group | Processing method after interruption | PCR product concentration (ng/µL) | Remarks (Fig. 4) |
|---|---|---|---|
| 1 | 2µL protease +1% Triton-X100 | 11.4 | Lane 1 |
| 2 | NT buffer +1% Triton-X100 | 13 | Lane 2 |
| 3 | 1% SDS +1% Triton-X100+0.5% Tween-20 | 12.4 | Lane 3 |
| 4 | 2µL protease +14mM EDTA+1% Triton-X100 | 12 | Lane 4 |
| 5 | 1 ×PBI, 1.3×Ampure XP beads | 13.5 | Lane 5 |
| 6 | 0.1µL protease +1mM EDTA+0.1 % Triton-X 100 | 6.2 | - |
| 7 | 5µL protease +50mM EDTA+2% Triton-X100 | 10.3 | - |
| 8 | 0.01% SDS+0.1% Triton-X100+0.1% Tween-20 | 5.3 | - |
| 9 | 1.5% SDS+2% Triton-X100+2% Tween-20 | 9.1 | - |
| 10 | 0.1µL protease +0.1% Triton-X100 | 6 | - |
| 11 | 5µL protease +2% Triton-X100 | 10.1 | - |

## Claims

1. A method for breaking a nucleic acid and adding an adapter by means of a transposase, comprising the following steps:
randomly interrupting a nucleic acid by using a transposase-embedded complex, wherein the transposase-embedded complex comprises a transposase and a first adapter comprising a transposase identification sequence, and both ends of the interrupted nucleic acid are separately ligated to the first adapter to form a gap at each end, wherein the first adapter is a double-stranded adapter;
eliminating the transposase in the system by means of purification, or by dissociating the transposase from a target sequence by denaturing or digesting the transposase by means of chemical reagent treatment;
ligating to a second adapter at the gap by using a ligase, wherein the sequence of the second adapter is different from that of the first adapter, wherein the second adapter having a modification preventing self-ligation, and the modification on the second adapter is a 3' terminal base dideoxy modification, wherein the second adapter is a double-stranded adapter; and
performing a PCR reaction by using primers targeted to the first adapter and the second adapter respectively, so as to obtain a product whose both ends are respectively ligated to different adapter sequences ;
wherein the first adapter has a modification to prevent self-ligation and inter-ligation with the second adapter , wherein the modification on the first adapter is a 3' terminal base dideoxy modification.

2. The method of claim 1 wherein the modification on the first adapter also comprises any one of the following or combination thereof:
(a) introducing a dUTP into a chain of the first adapter for subsequent enzymatic cleavage of excess adapters; and
(b) introducing a base pair at the outside of the transposase identification sequence of the first adapter.

3. The method of claim 1 wherein one of the primers used in the PCR reaction is a terminal biotin-labeled primer.

4. The method of any one of claims 1 to 3 wherein the purification is purification by magnetic beads or a column.

5. The method of any one of claims 1 to 3 wherein the chemical reagent comprises a first reagent and a second reagent; wherein the first reagent comprises one or more members of the group consisting of a protease solution and a SDS solution for breaking the adsorption effect of the transposase and the target sequence of the nucleic acid; and the second reagent comprises a Triton-XlOO solution for weakening the influence of the first reagent on the subsequent enzymatic reactions.

6. The method of claim 5 wherein the first reagent further comprises an additional reagent containing EDTA.

7. The method of claim 6, wherein the second reagent further comprises a Tween-20 solution.

## Patentansprüche

1. Verfahren zum Aufbrechen einer Nukleinsäure und Zufügen eines Adapters mittels einer Transposase, umfassend die folgenden Schritte:
zufälliges Unterbrechen einer Nukleinsäure mithilfe eines Transposase-eingebetteten Komplexes, wobei der Transposase-eingebettete Komplex eine Transposase und einen ersten Adaptor enthält, umfassend eine Transposase-Identifikationssequenz, und beide Enden der unterbrochenen Nukleinsäure zur Bildung einer Lücke getrennt an den ersten Adaptor an jedem Ende ligiert werden, wobei der erste Adaptor ein doppelsträngiger Adaptor ist;
Eliminieren der Transposase in dem System mittels Reinigung, oder durch Dissoziation der Transposase aus einer Zielsequenz durch Denaturierung oder Aufschluss der Transposase mittels einer Behandlung mit einem chemischen Reagenz;
Ligieren an einen zweiten Adaptor an der Lücke mithilfe einer Ligase, wobei die Sequenz des zweiten Adaptors sich von der des ersten Adaptors unterscheidet, wobei der zweite Adaptor eine Selbstligation verhindernde Modifikation aufweist, und die Modifikation an dem zweiten Adaptor eine 3'-terminale Basen-Didesoxy-Modifikation ist, wobei der zweite Adaptor ein doppelsträngiger Adaptor ist; und
Durchführen einer PCR-Reaktion mithilfe von an den ersten Adaptor bzw. an den zweiten Adaptor targetierten Primern, um auf diese Weise ein Produkt zu erhalten, dessen beide Enden jeweils an verschiedene Adaptorsequenzen ligiert sind;
wobei der erste Adaptor eine Modifikation zur Verhinderung einer Selbstligation und Zwischenligation mit dem zweiten Adaptor aufweist, wobei die Modifikation an dem ersten Adapter eine 3'-terminale Basen-Didesoxy-Modifikation ist.

2. Verfahren nach Anspruch 1, wobei die Modifikation an dem ersten Adaptor auch eines des Folgenden oder eine Kombination davon umfasst:
(a) Einführen eines dUTP in eine Kette des ersten Adaptors für die anschließende enzymatische Spaltung von überschüssigen Adaptoren; und
(b) Einführen eines Basenpaars an der Außenseite der Transposase-Identifikationssequenz des ersten Adaptors.

3. Verfahren nach Anspruch 1, wobei einer der in der PCR-Reaktion verwendeten Primer ein terminaler Biotinmarkierter Primer ist.

4. Verfahren nach einem der Ansprüche 1 bis 3, wobei die Reinigung eine Reinigung mittels magnetischer Beads oder einer Säule ist.

5. Verfahren nach einem der Ansprüche 1 bis 3, wobei das chemische Reagenz ein erstes Reagenz und ein zweites Reagenz umfasst; wobei das erste Reagenz ein oder mehr Mitglied(er) der Gruppe umfasst, bestehend aus einer Proteaselösung und einer SDS-Lösung zum Brechen des Adsorptionseffekts der Transposase und der Zielsequenz der Nukleinsäure; und das zweite Reagenz eine Triton X-100-Lösung zum Abschwächen des Einflusses des ersten Reagenzes auf die anschließenden enzymatischen Reaktionen umfasst.

6. Verfahren nach Anspruch 5, wobei das erste Reagenz ferner ein zusätzliches EDTA-enthaltendes Reagenz, umfasst.

7. Verfahren nach Anspruch 6, wobei das zweite Reagenz ferner eine Tween 20-Lösung umfasst.

## Revendications

1. Procédé destiné à rompre un acide nucléique et à ajouter un adaptateur au moyen d'une transposase, comprenant les étapes suivantes :
interruption aléatoire d'un acide nucléique en utilisant un complexe incrusté de transposase, dans lequel le complexe incrusté de transposase comprend une transposase et un premier adaptateur comprenant une séquence d'identification de transposase, et les deux extrémités de l'acide nucléique interrompu sont ligaturées séparément au premier adaptateur pour former un écartement au niveau de chaque extrémité, dans lequel le premier adaptateur est un adaptateur double brin ;
élimination de la transposase dans le system au moyen de la purification, ou en dissociant la transposase d'une séquence cible par dénaturation ou digestion de la transposase au moyen d'un traitement avec un réactif chimique ;
ligation d'un deuxième adaptateur au niveau de l'écartement en utilisant une ligase, dans lequel la séquence du deuxième adaptateur est différente de celle du premier adaptateur, dans lequel le deuxième adaptateur a une modification empêchant l'auto-ligation, et la modification sur le deuxième adaptateur est une modification didésoxy de base 3'-terminale, dans lequel le deuxième adaptateur est un adaptateur double brin ; et
réalisation d'une réaction de PCR en utilisant des amorces ayant pour cible le premier adaptateur et le deuxième adaptateur respectivement, de manière à obtenir un produit dont les deux extrémités sont respectivement ligaturées à des séquences d'adaptateur différentes ;
dans lequel le premier adaptateur comporte une modification pour prévenir l'auto-ligation et l'inter-ligation avec le deuxième adaptateur, dans lequel la modification sur le premier adaptateur est une modification didésoxy de base 3'-terminale.

2. Procédé selon la revendication 1 dans lequel la modification sur le premier adaptateur comprend aussi l'un quelconque des éléments suivants ou une combinaison de ceux-ci :
(a) introduction d'une dUTP dans une chaîne du premier adaptateur pour le clivage enzymatique subséquent d'adaptateurs excédentaires ; et
(b) introduction d'une paire de bases à l'extérieur de la séquence d'identification de transposase du premier adaptateur.

3. Procédé selon la revendication 1 dans lequel l'une des amorces utilisées dans la réaction de PCR est une amorce marquée avec une biotine terminale.

4. Procédé selon l'une quelconque des revendications 1 à 3 dans lequel la purification est une purification par des billes magnétiques ou une colonne.

5. Procédé selon l'une quelconque des revendications 1 à 3 dans lequel le réactif chimique comprend un premier réactif et un deuxième réactif ; dans lequel le premier réactif comprend un ou plusieurs éléments du groupe constitué d'une solution de protéase et d'une solution de SDS pour rompre l'effet d'adsorption de la transposase et la séquence cible de l'acide nucléique ; et le deuxième réactif comprend une solution de Triton-X100 destinée à affaiblir l'influence du premier réactif sur les réactions enzymatiques subséquentes.

6. Procédé selon la revendication 5 dans lequel le premier réactif comprend en outre de l'EDTA contenant un réactif supplémentaire.

7. Procédé selon la revendication 6, dans lequel le deuxième réactif comprend en outre une solution de Tween-20.


### SEQUENCE LISTING

<110> BGI SHENZHEN CO., LIMITED
<120> METHOD FOR BREAKING NUCLEIC ACID AND ADDING ADAPTOR BY MEANS OF
   TRANSPOSASE, AND REAGENT
<130> P49118EP-PCT
<140> EP14903871.3
   <141> 2014-10-14
<160> 20
<170> PatentIn version 3.3
<210> 1
   <211> 19
   <212> DNA
   <213> Artificial sequence
<220>
   <223> Sequence A of single-adapter 1
<220>
   <221> modified_base
   <222> (19)..(19)
   <223> dideoxy modification, 3'end dideoxy modification
<400> 1
   ctgtcuctta uacacatct 19
<210> 2
   <211> 34
   <212> DNA
   <213> Artificial sequence
<220>
   <223> Sequence B of single-adapter 1
<400> 2
   gcttcgactg gagacagatg tgtataagag acag 34
<210> 3
   <211> 20
   <212> DNA
   <213> Artificial sequence
<220>
   <223> Sequence A of single-adapter 2
<220>
   <221> modified_base
   <222> (20)..(20)
   <223> dideoxy modification
<400> 3
   gctgtctctt atacacatct 20
<210> 4
   <211> 35
   <212> DNA
   <213> Artificial sequence
<220>
   <223> Sequence B of single-adapter 2
<220>
   <221> modified_base
   <222> (35)..(35)
   <223> dideoxy modification, 3'end dideoxy modification
<400> 4
   gcttcgactg gagacagatg tgtataagag acagc 35
<210> 5
   <211> 53
   <212> DNA
   <213> Artificial sequence
<220>
   <223> Sequence of single-adapter 3
<220>
   <221> modified_base
   <222> (53)..(53)
   <223> dideoxy modification,3'end dideoxy modification
<400> 5
   gcttcgactg gagacagatg tgtataagag acagctgtct cttatacaca tct 53
<210> 6
   <211> 19
   <212> DNA
   <213> Artificial sequence
<220>
   <223> Sequence A of double-adapters
<400> 6
   ctgtctctta tacacatct 19
<210> 7
   <211> 33
   <212> DNA
   <213> Artificial sequence
<220>
   <223> Sequence B of double-adapters
<400> 7
   tcgtcggcag cgtcagatgt gtataagaga cag 33
<210> 8
   <211> 34
   <212> DNA
   <213> Artificial sequence
<220>
   <223> Sequence C of double-adapters
<400> 8
   gtctcgtggg ctcggagatg tgtataagag acag 34
<210> 9
   <211> 23
   <212> DNA
   <213> Artificial sequence
<220>
   <223> Sequence A of the NO.2 adapter
<220>
   <221> modified_base
   <222> (1)..(1)
   <223> phosphorylation modification, 5'end phosphorylation modification
<220>
   <221> modified_base
   <222> (23)..(23)
   <223> dideoxy modification, 3'end dideoxy modification
<400> 9
   aagtcggagg ccaagcggtc gtc 23
<210> 10
   <211> 14
   <212> DNA
   <213> Artificial sequence
<220>
   <223> Sequence B of the No.2 adapter
<220>
   <221> modified_base
   <222> (14)..(14)
   <223> dideoxy modification, 3'end dideoxy modification
<400> 10
   ttggcctccg actt 14
<210> 11
   <211> 41
   <212> DNA
   <213> Artificial sequence
<220>
   <223> Primer 1 of single-adapter
<400> 11
   agacaagctc gagctcgagc gatcgggctt cgactggaga c 41
<210> 12
   <211> 25
   <212> DNA
   <213> Artificial sequence
<220>
   <223> Primer 2 of single-adapter
<400> 12
   tcctaagacc gcttggcctc cgact 25
<210> 13
   <211> 20
   <212> DNA
   <213> Artificial sequence
<220>
   <223> Primer 1 of double-adapters
<400> 13
   aatgatacgg cgaccaccga 20
<210> 14
   <211> 21
   <212> DNA
   <213> Artificial sequence
<220>
   <223> Primer 2 of double-adapters
<400> 14
   caagcagaag acggcatacg a 21
<210> 15
   <211> 24
   <212> DNA
   <213> Artificial sequence
<220>
   <223> Mediating sequence for single-adapter
<400> 15
   tcgagcttgt cttcctaaga ccgc 24
<210> 16
   <211> 24
   <212> DNA
   <213> Artificial sequence
<220>
   <223> Mediating sequence for double-adapters
<400> 16
   cgccgtatca ttcaagcaga agac 24
<210> 17
   <211> 33
   <212> DNA
   <213> Artificial sequence
<220>
   <223> Sequence A of the NO.1 adapter in the form single-adapter
<400> 17
   tcgtcggcag cgtcagatgt gtataagaga cag 33
<210> 18
   <211> 19
   <212> DNA
   <213> Artificial sequence
<220>
   <223> Sequence B of the NO.1 adapter in the form of single-adapter
<220>
   <221> modified_base
   <222> (19)..(19)
   <223> dideoxy modification, 3'end dideoxy modification
<400> 18
   ctgtctctta tacacatct 19
<210> 19
   <211> 59
   <212> DNA
   <213> Artificial sequence
<220>
   <223> Primer 1 of the NO.1 adapter in the form of single-adapter
<400> 19
   agacaagctc gagctcgagc gatcgggatc tacacgactc actgatcgtc ggcagcgtc 59
<210> 20
   <211> 25
   <212> DNA
<220>
   <223> Primer 2 of the NO.1 adapter in the form of single-adapter
<400> 20
   tcctaagacc gcttggcctc cgact 25